# EUROPEAN PATENT APPLICATION

(11) **EP 2 779 143 A1**
(43) Date of publication of application: **17.09.2014**
(21) Application number: 11875567.7
(22) Date of filing: 10.11.2011
(51) Int. Cl.: G09B 23/28, A61B 1/00, A61B 8/12

(54) **TRAINING MODEL FOR ULTRASONIC BRONCHOSCOPY**

(71) Applicant: Koken Co. Ltd., Tokyo 112-0004 (JP)
(72) Inventor: SATO, Ryo, Tsuruoka-shi Yamagata 997-0011 (JP); TAKAHASHI, Futoshi, Tsuruoka-shi Yamagata 997-0011 (JP); IGARASHI, Masahiro, Tsuruoka-shi Yamagata 997-0011 (JP); MUKAI, Daisuke, Tsuruoka-shi Yamagata 997-0011 (JP)
(74) Representative: De Clercq, Ann G. Y.
(86) International application number: PCT/JP2011/076001
(87) International publication number: WO 2013/069143

(57) **Abstract**

Provided is a bronchoscopy training model for training in a technique for puncturing a lymph node adjacent to trachea/bronchi from the inside of the trachea/bronchi with an ultrasonic bronchoscope. Specifically, provided is a training model for training in ultrasound-guided needle puncture of a lymph node in a vicinity of trachea/bronchi, the training model including a jaw model, a bronchus model, and a replaceable needle puncture site, in which: the needle puncture site includes a simulated tracheal/bronchial cartilage, a simulated lymph node, and a simulated surrounding tissue; the simulated tracheal/bronchial cartilage, the simulated lymph node, and the simulated surrounding tissue in the needle puncture site each include as a main component any one of a silicone rubber and a urethane resin, and at least the simulated tracheal/bronchial cartilage and the simulated surrounding tissue in the needle puncture site each further include an organic powder filler blended therein; and the simulated tracheal/bronchial cartilage in the needle puncture site is provided on a tracheal/bronchial inner wall surface of the needle puncture site.

## Description

### Technical Field

The present invention relates to a training model for ultrasonic endoscopy, and more particularly, to a training model for training in ultrasound-guided needle puncture of a lymph node in the vicinity of trachea/bronchi.

### Background Art

Ultrasonic examination is indispensable in modern medicine. In particular, an ultrasonic endoscope, in which an ultrasonic probe is attached to an insertion tip, is a very useful device for pathological examination because the ultrasonic endoscope enables high-resolution ultrasonic observation, and moreover, enables a technique called endoscopic ultrasound-guided puncture, which involves collecting cells while performing ultrasonic observation for pathological examination.

Such ultrasonic examination requires high levels of knowledge, experience, and skill for a person who performs the examination. In particular, the endoscopic ultrasound-guided puncture technique requires additionally high levels of knowledge, experience, and skill.

In addition, in education and training for a person who performs the ultrasonic examination, the person is trained while examining actual patients under an instructor who has extensive knowledge and experience in a medical institution such as a hospital. Thus, there are considerable restrictions on a time for practical training and a disease to be experienced.

Therefore, there is a proposal of a human phantom for practical training in ultrasonic medicine for performing training in ultrasonic examination, which reproduces a shape or internal structure of a human body organ, and there is also a proposal of a human phantom including a replaceable puncture training site (see, for example, Patent Literature 1).

However, in the related-art model for training, training in a puncture technique has been performed only for a puncture site from esophagus or the like to an organ with no shielding.

In recent years, an ultrasonic bronchoscope has started to be heavily used for pathological diagnosis of tracheal/bronchial surroundings. However, puncture of a lymph node adjacent to trachea/bronchi with the ultrasonic bronchoscope has been limited to puncture at a position between rings of tracheal/bronchial cartilage, and hence has required a high level of puncture skill.

Therefore, in the related-art puncture training model, it has been impossible to sufficiently perform training in a technique for puncturing a lymph node adjacent to trachea/bronchi to be performed from the inside of the trachea/bronchi. Accordingly, there has been a strong demand for a training model adapted for such technique.

### Citation List

### Patent Literature

[PTL 1] JP 2004-174171 A

### Summary of Invention

### Technical Problem

The present invention has been made in order to solve the above-mentioned problems, and an object of the present invention is to provide an ultrasonic bronchoscopy training model for training in a technique for puncturing a lymph node adjacent to trachea/bronchi from the inside of the trachea/bronchi with an ultrasonic bronchoscope.

### Solution to Problem

A training model for ultrasonic bronchoscopy according to one embodiment of the present invention includes a training model for training in ultrasound-guided needle puncture of a lymph node in a vicinity of trachea/bronchi, in which: the training model includes a jaw model, a bronchus model, and a replaceable needle puncture site; the needle puncture site includes a simulated tracheal/bronchial cartilage, a simulated lymph node, and a simulated surrounding tissue; the simulated tracheal/bronchial cartilage, the simulated lymph node, and the simulated surrounding tissue in the needle puncture site each include as a main component any one of a silicone rubber and a urethane resin, and at least the simulated tracheal/bronchial cartilage and the simulated surrounding tissue in the needle puncture site each further include an organic powder filler blended therein; and the simulated tracheal/bronchial cartilage in the needle puncture site is provided on one side surface serving as a simulated tracheal/bronchial inner wall surface of the needle puncture site.

The simulated tracheal/bronchial cartilage provided in the needle puncture site preferably has a ladder shape.

In addition, the organic powder filler preferably includes a nylon filler, and the needle puncture site preferably includes as a main component a silicone rubber.

The simulated lymph node in the needle puncture site preferably includes a silicone rubber and liquid paraffin, and the simulated surrounding tissue in the needle puncture site preferably includes a silicone rubber, a nylon filler, and liquid paraffin.

The needle puncture site preferably has a square pole shape in which the simulated tracheal/bronchial cartilage is provided on one side surface serving as a simulated tracheal/bronchial inner wall surface of the needle puncture site and the simulated lymph node having a spherical shape is provided in the needle puncture site.

The bronchus model in the training model is preferably provided with a needle puncture site mounting portion having a concavity for mounting the needle puncture site in which part of a tracheal/bronchial wall is opened in a rectangular shape.

### Advantageous Effects of Invention

According to one embodiment of the present invention, it is possible to perform the training in the technique for puncturing a lymph node adjacent to trachea/bronchi to be performed from the inside of the trachea/bronchi, which has not been able to be performed in the related-art puncture training model. This can contribute to improving the skill of a practitioner in the puncture technique.

### Brief Description of Drawings

FIG. 1 is a schematic view illustrating positions of lymph nodes in the vicinity of trachea/bronchi.
FIG. 2 is an overall schematic view of an ultrasonic bronchoscopy training model according to Examples of the present invention.
FIG. 3 is a schematic view of a needle puncture site mounting portion of the ultrasonic bronchoscopy training model according to Examples of the present invention.
FIG. 4 is a schematic view of a needle puncture site according to Examples of the present invention.
FIG. 5 is a cross-sectional view of FIG. 4.

### Description of Embodiments

A preferred embodiment of the present invention is described below in detail with reference to the drawings. This embodiment is one example for carrying out the present invention, and the present invention is by no means limited to this embodiment.

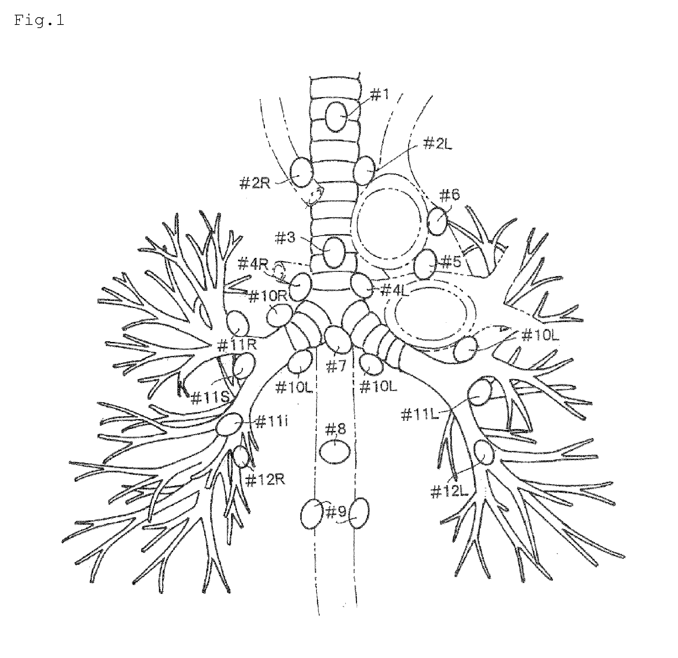

FIG. 1 illustrates locations of lymph nodes in the vicinity of trachea and bronchi. Of the lymph nodes #1 to #12 illustrated in FIG. 1, mediastinal lymph nodes (#1, #2, #3, #4, and #7) and hilar lymph nodes (#10, #11, and #12) are subjected to diagnosis with an ultrasonic bronchoscope, and the lymph nodes #5, #6, #8, and #9, which are not in contact with trachea or bronchi, are not subjected to the diagnosis.

A training model of the present invention is used for training in a technique for puncturing the above-mentioned lymph nodes in contact with trachea or bronchi.

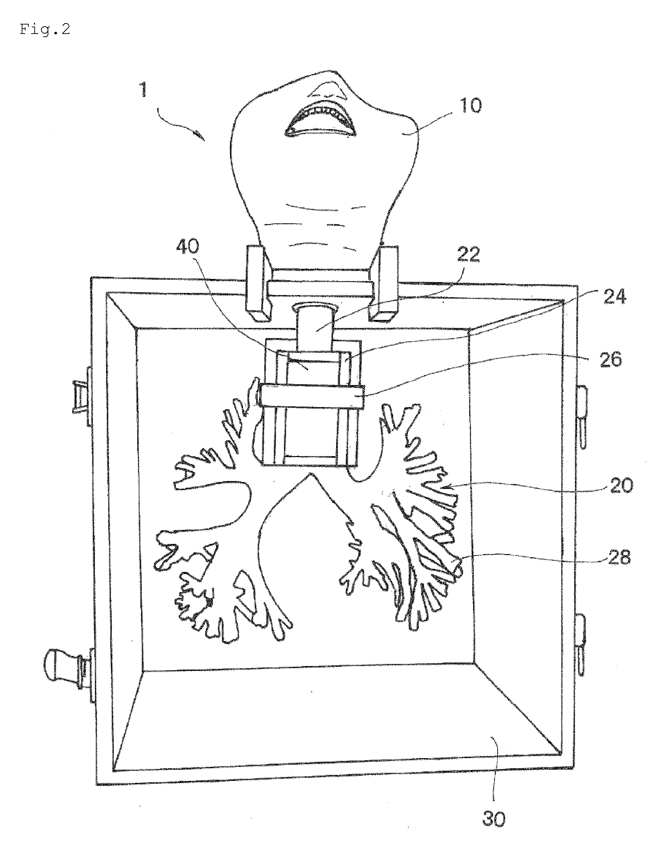

FIG. 2 is a schematic view of an ultrasonic bronchoscopy training model according to an embodiment of the present invention.

An ultrasonic bronchoscopy training model 1 according to an embodiment of the present invention includes a jaw model 10, a bronchus model 20, a case 30, and a needle puncture site 40.

The jaw model 10 in the embodiment of the present invention is a part serving as an insertion port at the time of the insertion of an ultrasonic bronchoscope. A jaw model made similar to an actual jaw is preferably used for making the situation similar to that in the case of performing the insertion in a human body to enhance a training effect.

In addition, the jaw model 10 is provided with a laryngeal portion (not shown) serving as an insertion port for an endoscope. The end of the laryngeal portion is connected to a simulated tracheal portion of the bronchus model.

The laryngeal portion of the jaw model is desirably made removable so as to be replaceable at the time of its breakage.

The bronchus model 20 in the embodiment of the present invention includes: a simulated tracheal portion 22, which is connected to the laryngeal portion of the jaw model 10; a needle puncture site mounting portion 24, which is provided on the upper side of a branched tracheal portion of the simulated tracheal portion 22; and a simulated bronchial portion 28, which is positioned on the lower side of the simulated tracheal portion 22.

A silicone rubber, a urethane resin, a vinyl chloride resin, or the like may be used as a material for each of the simulated tracheal portion 22 and the simulated bronchial portion 28 in the bronchus model 20. In particular, a silicone rubber is preferred used because a sensation to be obtained at the time of the insertion of the bronchoscope is a human body-like sensation.

In addition, the simulated bronchial portion 28, which reproduces minor bronchi as well, may be used not only for training in a puncture technique but also for general training in bronchoscopy. In that case, it is preferred to reproduce up to the fifth-order bronchi so that the simulated bronchial portion may also be used for training in ultrathin bronchoscopy.

The case 30, which is a case for accommodating the bronchus model 20, is opened on the front surface side so as to allow the observation of a state in which light for lighting from an endoscope tip can be seen through a simulated tracheal/bronchial wall. Thus, the position of the endoscope tip can be recognized. This is useful for enhancing the effect of bronchoscopy training.

In this embodiment, the needle puncture site 40 is mounted to the needle puncture site mounting portion 24 provided above the branched tracheal portion of the simulated tracheal portion 22. Each of the needle puncture site mounting portion 24 and the needle puncture site 40 is not necessarily set at a position on the upper side of the branched tracheal portion of the simulated tracheal portion 22, and may be provided in the simulated bronchial portion 28. The needle puncture site 40 is removably mounted to the needle puncture site mounting portion 24 so that the needle puncture site 40 damaged by repeating the puncture technique is easily replaceable.

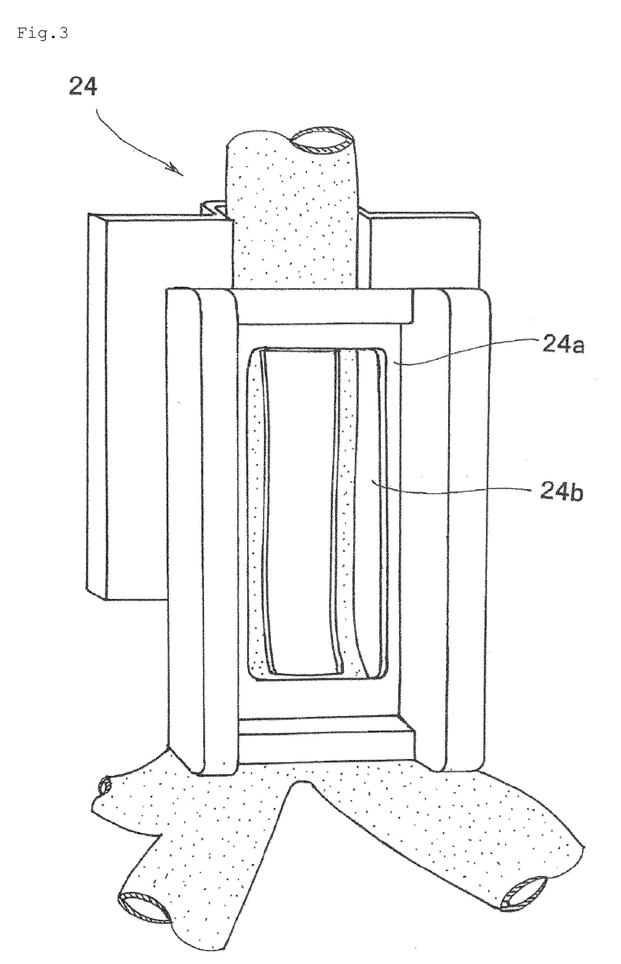

FIG. 3 is a schematic view of the needle puncture site mounting portion 24.

The needle puncture site mounting portion 24 is provided with a mounting concavity 24a having a concavity formed so as to fit the shape of the needle puncture site 40 in order to mount the needle puncture site 40.

The bottom portion of the mounting concavity 24a facing the simulated tracheal portion 22 side is provided with a puncture opening 24b in which part of the bottom surface of the concavity is opened. The puncture opening 24b plays a role as a window for applying the puncture technique to the mounted needle puncture site 40 from the inside of simulated trachea/bronchi with the bronchoscope.

In addition, the needle puncture site mounting portion 24 is provided with a fixing belt 26 for fixing the needle puncture site so that the needle puncture site 40 mounted to the needle puncture site mounting portion 24 is prevented from being demounted during training.

Any shape may be adopted as the shape of the mounting concavity 24a as long as the needle puncture site 40 can be accommodated. In addition, any shape may be adopted as the shape of the puncture opening 24b as long as the puncture technique is applicable. However, a rectangular shape along a vertical direction of the simulated tracheal portion or the simulated bronchial portion is preferred from the viewpoint of ease of puncture technique training.

Any shape may be adopted as the shape of the needle puncture site 40 as long as the puncture technique is applicable. However, like the shape of the puncture opening 24b, a square pole shape along a vertical direction of the simulated trachea is preferred from the viewpoints of ease of puncture technique training and ease of replacement. The shape in this embodiment is a square pole shape measuring 6 cm in height by 3 cm in width by 4 cm in depth, which allows easy replacement.

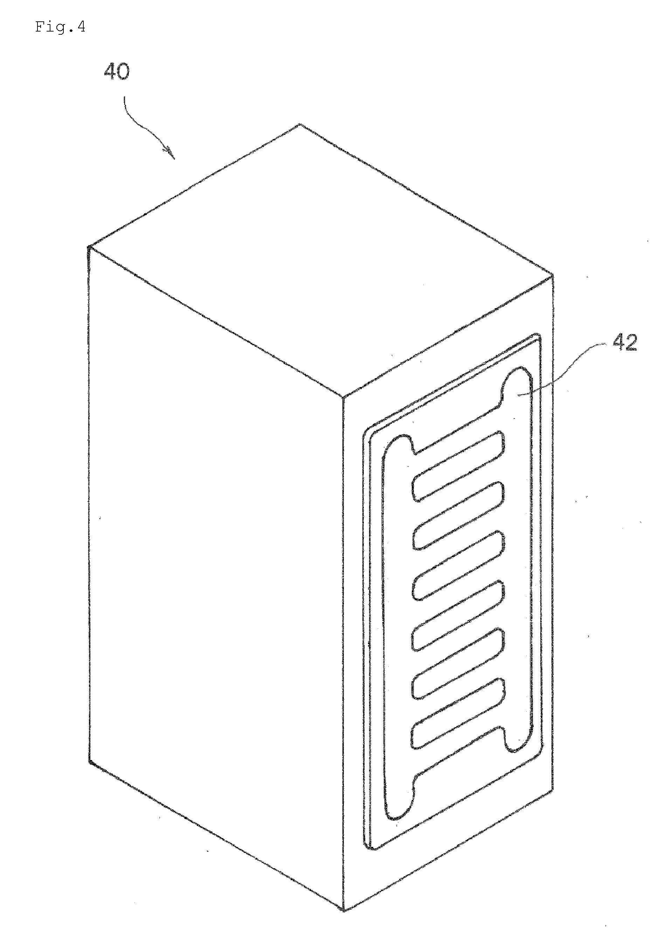

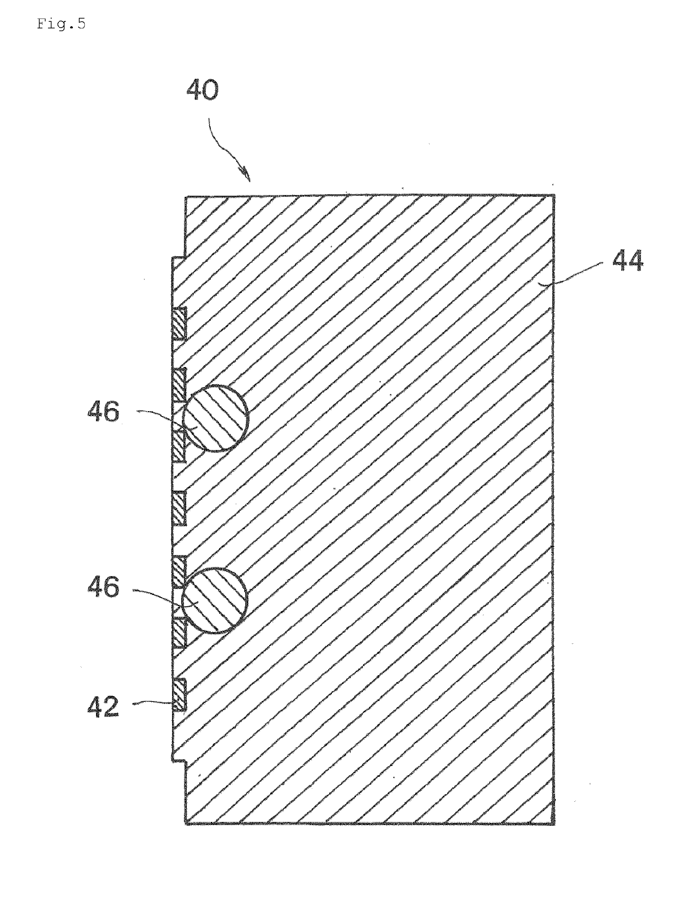

FIG. 4 is a schematic view of the needle puncture site 40 according to the embodiment of the present invention, and FIG. 5 is a cross-sectional view of FIG. 4.

As illustrated in FIG. 4 and FIG. 5, the needle puncture site 40 includes a simulated tracheal/bronchial cartilage 42, a simulated surrounding tissue 44, and simulated lymph nodes 46, and has such a construction that: the simulated tracheal/bronchial cartilage 42 is provided on one side surface serving as a simulated tracheal/bronchial inner wall surface of the simulated surrounding tissue 44; and the simulated lymph nodes 46 are provided in the simulated surrounding tissue 44.

The simulated tracheal/bronchial cartilage 42 is provided on one side surface serving as a simulated tracheal/bronchial inner wall surface of the needle puncture site 40 so as to fit the puncture opening 24b provided at the bottom portion of the mounting concavity 24a of the needle puncture site mounting portion 24, and forms part of the simulated tracheal portion or simulated bronchial portion inner wall.

It is preferred that the shape of the simulated tracheal/bronchial cartilage 42 be made similar to the shape of an actual tracheal cartilage in a human body and have a ladder shape. Specifically, as illustrated in FIG. 4, the shape is such a shape that lateral portions extend at a predetermined interval from longitudinal portions formed along trachea/bronchi, and portions in which the simulated surrounding tissue 44 is exposed between the lateral portions for imitating the tracheal/bronchial cartilage correspond to interchondral ligaments.

In addition, in order to enhance the training effect, the simulated tracheal/bronchial cartilage 42 preferably has a thickness similar to that of an actual tracheal/bronchial cartilage, and preferably has a thickness of from 1.5 mm to 2.0 mm.

When the training model according to the embodiment of the present invention is used for training in a technique for puncturing a lymph node adjacent to trachea/bronchi from the inside of trachea/bronchi with an ultrasonic bronchoscope, needle insertion is first performed for the portions corresponding to interchondral ligaments in which the simulated surrounding tissue 44 is exposed between the lateral portions for imitating the tracheal/bronchial cartilage, and then the simulated lymph nodes 46 present on the back side of the simulated tracheal/bronchial cartilage are punctured. The adoption of the above-mentioned shape as the shape of the simulated tracheal/bronchial cartilage 42 is useful for improving the skill of puncturing a lymph node from between the lateral portions of the tracheal/bronchial cartilage during puncture technique training.

In the training model for ultrasonic bronchoscopy according to the embodiment of the present invention, a trainee inserts an ultrasonic bronchoscope into the simulated tracheal portion 22 from the laryngeal portion of the jaw model 10, and applies the puncture technique to the simulated lymph nodes 46 on the back side of the simulated tracheal/bronchial cartilage 42 through the simulated surrounding tissue 44 at a gap for imitating that between rings of the tracheal/bronchial cartilage. Therefore, the simulated tracheal/bronchial cartilage 42, the simulated surrounding tissue 44, and the simulated lymph nodes 46 need to be designed so as to provide an ultrasonic image similar to that in an actual human body and to allow the borders to be clearly distinguished from each other.

In addition, a material for each of the simulated tracheal/bronchial cartilage 42, the simulated surrounding tissue 44, and the simulated lymph nodes 46 in the needle puncture site 40 is selected so that a texture upon puncture is a human body-like texture.

The simulated tracheal/bronchial cartilage 42, the simulated surrounding tissue 44, and the simulated lymph nodes 46 in the needle puncture site 40 are each formed by using any one of a silicone rubber and a urethane resin as a main component. In particular, a silicone rubber is preferred from the viewpoints of ease of adjustment of an ultrasonic image and resistance to repeated puncture, a liquid silicone rubber is more preferred from the viewpoint of ease of cross-linking and hardness adjustment, and a two-component addition type silicone rubber, which does not generate any condensed product at the time of curing, is still more preferred.

In addition, in at least the simulated tracheal/bronchial cartilage 42 and the simulated surrounding tissue 44 in the needle puncture site 40, an organic powder filler is blended in the above-mentioned main component material. The kind, shape, and amount of the organic powder filler to be blended are appropriately selected so as to provide an ultrasonic image similar to that in an actual human body. The use of a nylon filler as the organic powder filler is preferred from the viewpoints of ease with which an ultrasonic image is made similar to that in an actual human body and ease with which the contrast between simulated organs is provided.

In this embodiment, the simulated tracheal/bronchial cartilage 42 is preferably formed of a silicone rubber blended with a nylon filler. The hardness of the silicone rubber to be used in the simulated tracheal/bronchial cartilage 42 is preferably set higher than those of the simulated surrounding tissue 44 and the simulated lymph nodes 46 so that a puncture texture is a human body-like texture.

In this embodiment, in the simulated surrounding tissue 44 and the simulated lymph nodes 46, a lubricant is preferably mixed into the main component material for hardness adjustment and an improvement in needle penetration property so that a texture upon puncture is a human body-like texture.

The lubricant to be mixed into each of the simulated surrounding tissue 44 and the simulated lymph nodes 46 is preferably a liquid lubricant from the viewpoint of ease of hardness adjustment. In particular, liquid paraffin is preferred.

The amount of the lubricant to be mixed into each of the simulated surrounding tissue 44 and the simulated lymph nodes 46 is preferably from 15 wt% to 25 wt% with respect to the main component material. When the amount is controlled within the range, such an effect that a sensation upon puncture becomes a human body-like texture is exhibited.

The positions of the simulated lymph nodes 46 in the simulated surrounding tissue 44 are made similar to those of the actual lymph nodes, and the simulated lymph nodes are formed at positions in proximity to the simulated tracheal/bronchial cartilage 42 in the simulated surrounding tissue 44. The shape of each of the simulated lymph nodes 46 is preferably a spherical shape having a diameter of from 5 mm to 10 mm, and the number of the simulated lymph nodes to be formed in the needle puncture site 40 is preferably from 2 to 6.

In this embodiment, the position to which the needle puncture site 40 is mounted is trachea. Thus, of the lymph nodes illustrated in FIG. 1, 2 to 6 simulated lymph nodes corresponding to #1, #2 (R and L), #3, and #4 (R and L) are provided as the simulated lymph nodes 46.

### Reference Signs List

- 1: bronchoscopy training model
- 10: jaw model
- 20: bronchus model
- 22: simulated tracheal portion
- 24: needle puncture site mounting portion
- 24a: mounting concavity
- 24b: puncture opening
- 26: fixing belt
- 28: simulated bronchial portion
- 30: case
- 40: needle puncture site
- 42: simulated tracheal/bronchial cartilage
- 44: simulated surrounding tissue
- 46: simulated lymph node

## Claims

1. A training model for ultrasonic bronchoscopy, comprising a training model for training in ultrasound-guided needle puncture of a lymph node in a vicinity of trachea/bronchi, wherein:
the training model includes a jaw model, a bronchus model, and a replaceable needle puncture site;
the needle puncture site includes a simulated tracheal/bronchial cartilage, a simulated lymph node, and a simulated surrounding tissue;
the simulated tracheal/bronchial cartilage, the simulated lymph node, and the simulated surrounding tissue in the needle puncture site each comprise as a main component any one of a silicone rubber and a urethane resin, and at least the simulated tracheal/bronchial cartilage and the simulated surrounding tissue in the needle puncture site each further comprise an organic powder filler blended therein; and
the simulated tracheal/bronchial cartilage in the needle puncture site is provided on a simulated tracheal/bronchial inner wall surface of the needle puncture site.

2. A training model for ultrasonic bronchoscopy according to claim 1, wherein the simulated tracheal/bronchial cartilage provided in the needle puncture site has a ladder shape.

3. A training model for ultrasonic bronchoscopy according to claim 1, wherein the organic powder filler comprises a nylon filler, and the needle puncture site comprises a silicone rubber.

4. A training model for ultrasonic bronchoscopy according to claim 1, wherein the simulated lymph node in the needle puncture site comprises a silicone rubber and liquid paraffin.

5. A training model for ultrasonic bronchoscopy according to claim 1, wherein the simulated surrounding tissue in the needle puncture site comprises a silicone rubber, a nylon filler, and liquid paraffin.

6. A training model for ultrasonic bronchoscopy according to claim 1, wherein the needle puncture site has a square pole shape in which the simulated tracheal/bronchial cartilage is provided on one side surface of the needle puncture site and the simulated lymph node having a spherical shape is provided in the needle puncture site.

7. A training model for ultrasonic bronchoscopy according to claim 1, wherein the bronchus model in the training model is provided with a needle puncture site mounting portion having a concavity for mounting the needle puncture site in which part of a tracheal/bronchial wall is opened in a rectangular shape.
